# EUROPEAN PATENT SPECIFICATION

(11) **EP 0 721 766 B1**
(45) Date of publication and mention of the grant of the patent: **03.02.1999**
(21) Application number: 95306489.6
(22) Date of filing: 14.09.1995
(51) Int. Cl.: A61B 17/22, A61M 25/01

(54) **Vascular incisor/dilator**
Vorrichtung zum Einschneiden und Aufdehnen von Blutgefässen
Dispositif d'incision et de dilatation vasculaire

(30) Priority: 10.01.1995 US 370893
(43) Date of publication of application: 17.07.1996
(73) Proprietor: INTERVENTIONAL TECHNOLOGIES INC, San Diego California 92123 (US)
(72) Inventor: Lary, Banning Gray, Miami, Florida 33143 (US)
(74) Representative: MacGregor, Gordon

(56) References cited:
- EP-A- 0 401 158
- EP-A- 0 419 154
- EP-A- 0 619 986
- WO-A-90/07909
- US-A- 4 867 156

## Description

### FIELD OF THE INVENTION

The present invention pertain generally to surgical devices. More particularly, the present invention pertains to devices for clearing a stenosis from the artery of a patient. The present invention is particularly, but not exclusively useful, for both incising and subsequently dilating a vessel to clear an obstruction or stenosis from the vessel.

### BACKGROUND OF THE INVENTION

Many medical complications are created by the total or even partial blockage of blood vessels of the body. The primary cause of these complications is, of course, the reduction or cessation of blood flow through the blocked vessels to the particular biological tissue which is serviced by the vessel. Most commonly, a blockage, or stenosis, is formed in an artery as a result of plaque build-up in the artery. Further, it is not uncommon for several stenoses to occur sequentially in a single artery or to develop near one another in branches of a common central artery.

Several methods, or procedures, have been developed in the medical field for the purpose of removing or clearing stenoses from the vessels of patients. One well known procedure for accomplishing this is an angioplasty procedure such as is disclosed in U.S. Patent No. Re. 33,561 which issued to Levy for an invention entitled "BALLOON AND MANUFACTURE THEREOF". Basically, in an angioplasty procedure, a deflated dilatation balloon is inserted into the vessel and is placed across the stenosis. Once the balloon is properly positioned, it is then inflated to dilate the artery and thereby clear the stenosis. Another, more recently developed procedure for clearing a stenosis, is an atherectomy procedure. The essential aspects of an atherectomy procedure are set forth in U.S. Patent No. 4,895,166 which issued to Farr et al. for an invention entitled "ROTATABLE CUTTER FOR THE LUMEN OF A BLOOD VESSEL". As disclosed by Farr et al., in an atherectomy procedure the stenotic material is actually cut and removed from the artery. Both the angioplasty procedure and the atherectomy procedure are typically accomplished indirectly wherein access to the stenosis is achieved through a peripheral artery. These procedures are in contrast to other known procedures used to clear arteries, such as a by-pass surgery, where direct access to the stenosis is achieved by entering the artery at or near the site of the stenosis. Despite their differences, the ultimate objective of all these procedures is to remove or alleviate the stenosis which is restricting blood flow through the artery.

Recent studies have indicated that for procedures wherein a stenosis is to be dilated, such as for an angioplasty procedure, the efficacy of the dilatation is enhanced by first incising the material which is creating the stenosis. With this knowledge, several devices for clearing blocked arteries have been proposed. For example, U.S. Patent No. 4,273,128 which issued to Lary for an invention entitled "CORONARY CUTTING AND DILATING INSTRUMENT" discloses a surgical instrument which both incises and dilates a stenosis. As another example, U.S. Patent No. 5,209,799 which issued to Vigil for an invention entitled "METHOD FOR MANUFACTURING A FOLDING BALLOON CATHETER" discloses a folding angioplasty balloon with attached atherotomes.

While angioplasty, atherectomy and by-pass surgery procedures, as disclosed above, are efficacious for their intended purposes, it happens that less aggressive methods may also be appropriate and just as effective for removing or clearing a stenosis from the vessel of a patient. Moreover, less aggressive methods may be preferable. This is particularly so where shorter, and perhaps more numerous, stenoses are involved.

EP-A-619 986 (Interventional Technologies), which forms the basis for the two-part form of claim 1 below, relates to an insertable catheter device which longitudinally incises a stenotic segment of an artery prior to balloon dilation. Generally, the catheter device includes a hollow catheter sheath and a cutting member which is reciprocatably mounted in the catheter sheath.

In light of the above it is an object of the present invention to provide a device for incising and dilating a stenosis in a vessel of a patient which is particularly efficacious for shorter stenotic blockages in the vessel. Another object of the present invention is to provide a device for incising and dilating a stenosis in a vessel of a patient which can be used with either direct or indirect access to the stenosis. Still another object of the present invention is to provide a device for incising and dilating a stenosis in a vessel of a patient which can be effectively used to augment a more aggressive procedure such as either angioplasty, atherectomy or by-pass surgery. Yet another object is to provide a device which can cut and dilate numerous areas of stenoses in a single vessel and another to provide and which is replaceable with a smaller identical apparatus which will pass through proximal stenoses that have been previously removed to clear stenoses in the smaller distal position of the same vessel. Yet another object of the present invention is to provide a device for incising and dilating a stenosis in a vessel of a patient which is relatively simple to manufacture, is easy to use, and is comparatively cost effective.

### SUMMARY OF THE INVENTION

The present invention provides a device for clearing a stenosis in a vessel of a patient according to the accompanying claims.

A device for incising and dilating a stenosis in a vessel of a patient includes a probe catheter and a probe member which is fixedly attached to the distal end of the probe catheter. The probe member is generally cone-shaped with smooth transitions and is tapered with diminishing cross section in the distal direction. Additionally, a plurality of atheretomes, or elongated blades, are mounted on the probe member and are aligned longitudinally with the probe catheter.

In the operation of the device of the present invention, the probe member is advanced through a vessel of the patient to the stenosis. At the stenosis, the probe member may be reciprocally moved back and forth through the stenosis, as necessary, to incise the stenosis with the blades and to dilate the stenosis with the tapered portion of the probe member. Several embodiments of the present invention are possible for advancing the probe member to the stenosis.

One embodiment of the present invention requires a contiguous lumen be formed through the probe catheter and the probe member. A guidewire can then be prepositioned in the vessel at least up to the stenosis and, with the guidewire inserted into the contiguous lumen, the combination of probe catheter and probe member can be advanced to the stenosis. In another embodiment of the present invention, a guide catheter is provided which has a lumen for receiving the combination of probe catheter and probe member therethrough. For this embodiment, the guide catheter is prepositioned in the vessel of the patient and the probe member is advanced through the lumen of the guide catheter to the stenosis.

The probe catheter can also include a dilatation balloon. For this embodiment of the present invention, a selectively inflatable balloon is incorporated into the probe catheter at a location on the probe catheter that is proximal to the probe member. In the operation of this embodiment, after the probe member has been advanced through the stenosis to incise and initially dilate the stenosis, the dilatation balloon is positioned across the stenosis and inflated for further dilatation of the stenosis.

Operation of the probe catheter for advancing the probe member to the stenosis and for moving the probe member through the stenosis can be facilitated with the incorporation of a rigidizer in the probe catheter. For purposes of the present invention, the rigidizer is located on the probe catheter immediately proximal to the probe member. The rigidizer includes a deformable cavity which is formed into the probe catheter to confine loosely packed granules, and a suction device for evacuating air from the cavity to tightly pack the granules together. Thus, with some air in the rigidizer cavity, the loosely packed granules allow for flexibility of the probe catheter during advancement of the probe member to the stenosis. On the other hand, upon evacuation of air from the rigidizer cavity, the granules become tightly packed and provide a stiff rigid structure for movement of the probe member through the stenosis.

In addition to improving the operability of the device for cleaning a stenosis, this feature also permits the apparatus to maneuver effectively through branches and angles of the blood vessels.

### BRIEF DESCRIPTION OF THE DRAWINGS

The novel features of this invention, as well as the invention itself, both as to its structure and its operation will be best understood from the accompanying drawings, taken in conjunction with the accompanying description, in which similar reference characters refer to similar parts, and in which:
Figure 1 is a perspective view of a patient being operated on, with the device of the present invention being indirectly inserted through a peripheral vessel of the patient to the stenosis site;
Figure 2 is a perspective view of the probe member of the device of the present invention shown in cooperation with a guidewire;
Figure 3 is an exemplary view of the probe member of the device of the present invention at a stenosis in a vessel of the patient;
Figure 4 is a perspective view of the device of the present invention being used with a guide catheter;
Figure 5A is a perspective view of an alternate embodiment of the device of the present invention which incorporates a dilatation balloon, with the balloon deflated;
Figure 5B is a perspective view of the device of the present invention, as seen in Figure 5A, with the dilatation balloon inflated;
Figure 6 is a cross sectional view of the device of the present invention as seen along the line 6-6 in Figure 5B;
Figure 7A is a cross sectional view of another embodiment of the device of the present invention which incorporates a rigidizing structure, as this embodiment would be seen along the line 6-6 in Figure 5B, with the rigidizing structure in a flexible configuration; and
Figure 7B is a cross sectional view of the embodiment of the device of the present invention shown in Figure 7A with the rigidizing structure in a rigid configuration.

### DESCRIPTION OF THE PREFERRED EMBODIMENTS

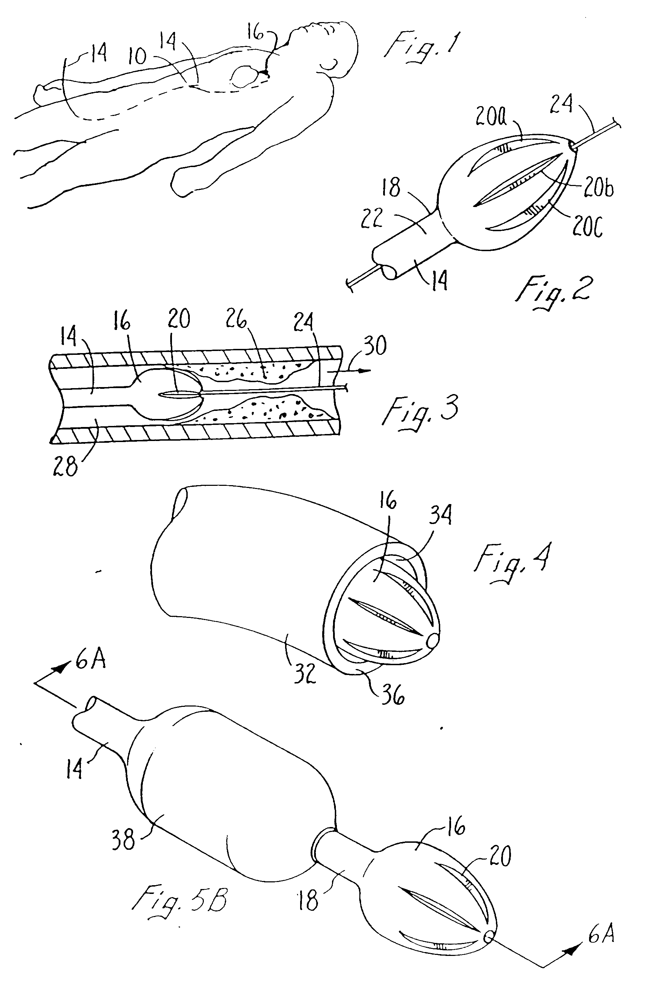

Referring initially to Figure 1 the device for incising and dilating a stenosis in a patient is shown and generally designated 10. As shown, the device 10 is shown inserted into a peripheral artery of the patient 12. The device 10 generally includes a probe catheter 14 and a probe member 16. As indicated in Figure 1, the probe catheter 14 is located proximal to the probe member 16, and the probe member 16 is fixedly attached to the distal end 18 of probe catheter 14 for movement therewith.

As perhaps best seen in Figure 2, the probe member 16 of the device 10 is generally cone shaped and is tapered with a diminishing cross section in the distal direction. Further, probe member 16 has smooth transitions so as not to present rough or angular edges which could become caught or snagged on irregularities in the vessel. Additionally, and importantly, probe member 16 includes a plurality of blades 20, of which the blades 20 a-c are exemplary. More specifically, the blades 20 are mounted on the tapered portion of probe member 16 and are aligned with a longitudinal axis that is defined by an extended probe catheter 14. Figure 2 also shows that both the probe catheter 14 and probe member 16 are formed with a contiguous lumen 22 which extends the entire length of the device 10. As will be appreciated by the skilled artisan, lumen 22 is dimensioned to receive a guidewire 24 therethrough to facilitate proper placement of the probe member 16 in the vessel of the patient 12.

As indicated in Figure 3, in the operation of the device 10, the guidewire 24 is prepositioned in the patient 12. The probe member 16 and probe catheter 14 are then positioned over the guidewire 24. This is done by inserting guide wire 24 into lumen 22 of the device 10. Probe member 16 is then advanced along the guidewire 24 and into contact with the stenosis 26 which is blocking blood flow through the vessel of the patient 12. Typically, and as shown in Figure 3, a stenosis 26 occurs due to the build-up of plaque on the wall 28 of the vessel. To clear the stenosis 26, the effect of the build-up must be removed or otherwise eliminated. With the device 10 of the present invention this is accomplished by advancing the probe member 16 along guide wire 24 in the direction of arrow 30 and through the stenosis 26. As this is done, the blade 20 of probe member 16 first incise the stenosis 26. Additional advancement of probe member 16 causes the probe member 16, itself, to urge against the stenosis 26. This causes the stenosis to dilate as probe member 16 is advanced. If necessary, probe member 16 can be moved back and forth across the stenosis 26 with multiple passes.

As an alternative for advancing probe member 16 to the site of the stenosis 26, rather than using a guidewire 24, a guide catheter 32 can be employed. As shown in Figure 4, guide catheter 32 is formed with a lumen 34 which is of sufficient size to receive the probe member 16 and probe catheter 14 therethrough. In the operation of this embodiment of the present invention, the guide catheter 32 is prepositioned in the vessel of patient 12 with its distal end 36 located just proximal to the stenosis 26. Probe member 16 is then advanced through lumen 34 of guide catheter 32 and into contact with stenosis 26. Again, as disclosed above, stenosis 26 is incised and dilated as probe member 16 is further advanced distally from distal end 36 of guide catheter 32 through stenosis 26. Also, as before, probe member 16 can be given a back and forth motion across the stenosis 26, if required.

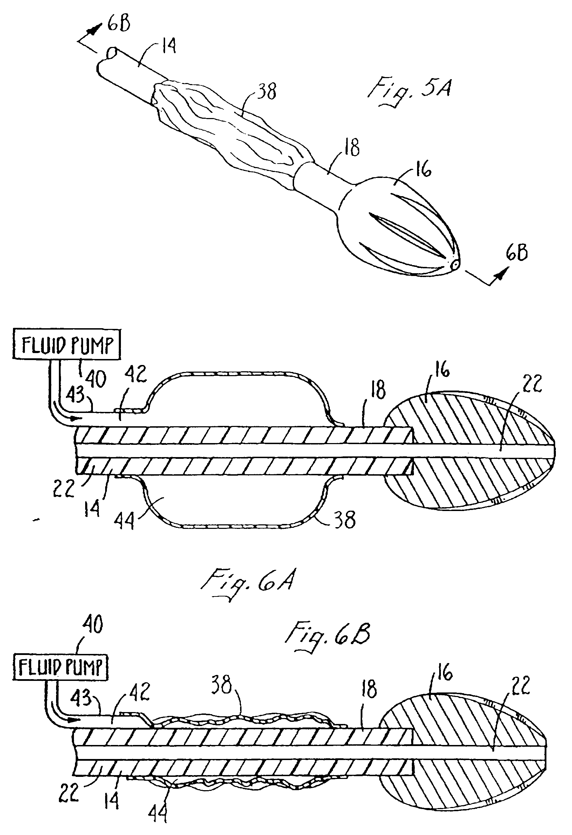

Figures 5A, 5B and 6 illustrate an alternate embodiment for the device 10 which additionally incorporates a dilatation balloon 38. More specifically, as shown, the dilatation balloon 38 is positioned on the probe catheter 14 at a location proximal to probe member 16. As best seen in Figure 6A, a fluid pump 40 which is located off the proximal end of probe catheter 14, and which remains extracorporeal, is connected in fluid communication with a passageway 42 that is formed by a tube 43 which is coaxial with probe catheter 14 and which is attached in a fluid communication with the proximal end of balloon 38. Accordingly, by selectively operating fluid pump 40, fluid can be introduced into the potential chamber 44 established by balloon 38 to change device 10 from the configuration shown in Figure 5A, wherein balloon 38 is deflated, into the configuration shown in Figure 5B, wherein balloon 38 is inflated. In the operation of this alternate embodiment of device 10, probe member 16 can be advanced to a stenosis 26 using either a guidewire 24 or a guide catheter 32 as disclosed above. Once probe member 16 has been advanced through stenosis 26 to incise stenosis 26 with blades 20 and dilate stenosis 26, a deflated balloon 38 can be positioned across the stenosis 26. Inflation of balloon 38 at that point will then provide further action for dilating stenosis 26. When desired, balloon 38 can be deflated and the device 10 removed from the vessel of patient 12.

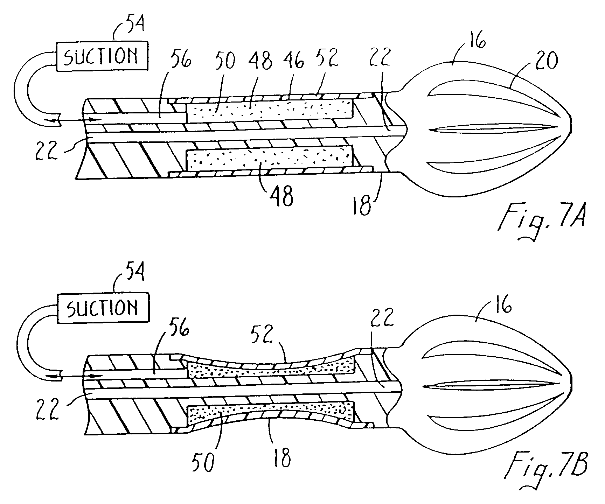

Yet another embodiment for the device 10 of the present invention is shown in Figures 7A and 7B. This particular embodiment incorporates a rigidizer 46 which allows the distal end 18 of probe catheter 14 to be selectively either limp or rigid. More specifically, rigidizer 46 includes a chamber 48 that is formed into the shaft of probe catheter 14 and which is loosely filled with biocompatible granules 50 (such as particles of dextran). More specifically, the external wall of the chamber 48 can include a sleeve 52 which is made of a relatively flexible material. The sleeve 52 is attached to probe catheter 14 to cover chamber 48. It will be appreciated, that probe catheter 14 is, itself, made of a somewhat flexible material. Sleeve 52, however, can be even more flexible or even deformable. Figure 7A also shows that a suction pump 54 is located off the proximal end of probe catheter 14 and that the suction pump 54 is in fluid communication with a lumen 56 which is formed in probe catheter 14. Lumen 56, in turn, is in fluid communication with chamber 48.

As intended for the present invention, when the rigidizer 46 is deactivated, the granules 50 are loosely held in chamber 48. Consequently, with a deactivated rigidizer 46 the probe member 16 is effectively held by only the flexible material of probe catheter 14. This allows the distal end 18 of probe catheter 14 and the probe member 16 to be limp and therefore more maneuverable in their advancement toward a stenosis 26. This advancement may, of course, be either over a guidewire 24 or through a guide catheter 32. On the other hand, such limpness most likely will not be suitable for a further advancement of probe member 16 through the stenosis 26. To do this, the rigidizer 46 needs to be activated.

Activation of rigidizer 46 is accomplished by pulling a vacuum with the suction pump 54 to evacuate air or fluid from the chamber 48. Due to the flexibility of sleeve 52, as air or fluid is evacuated from chamber 48 the sleeve 52 will collapse under the action of the suction pump 54. This, in turn, will cause granules 50 in chamber 48 to be compressed onto each other forming a rigid structure. The result is that that portion of probe catheter 14 where chamber 48 is located becomes stiff and rigid, holding whatever shape the catheter was in when the tube was evacuated. This stiffness then allows the user to advance probe member 16 through a stenosis 26 without a reduced risk of any buckling or bending of the distal end 18 of probe catheter 14.

Although the device 10 has been described and disclosed for cooperation with a guidewire 24 or guide catheter 32, it is to be appreciated that the device 10 can be operated without either the guidewire 24 or guide catheter 32. This will particularly be the case when direct access to a stenosis is possible. Without a guidewire 24 or guide catheter 32, the probe catheter 14 must, itself, have sufficient controllability and steerability to advance the probe member 16 to a stenosis.

While the particular device for incising and dilating a stenosis in a vessel of a patient as herein shown and disclosed in detail is fully capable of obtaining the objects and providing the advantages herein before stated, it is to be understood that it is merely illustrative of the presently preferred embodiments of the invention and that no limitations are intended to the details of the construction or design herein shown other than as defined in the appended claims.

## Claims

1. A device for clearing a stenosis in a vessel of a patient which comprises:
means for incising the stenosis (26);
a probe member (16) for dilating the stenosis (26); and
means for reciprocally moving the probe member (16) and said incising means through the stenosis (26) to incise and dilate the stenosis (26), the probe member (16) being fixedly attached to the moving means; characterised in that said incising means is a plurality of blades (20) fixedly attached to said probe member (16) and extending substantially distally therefrom.

2. A device according to claim 1 further comprising means for advancing said incising means and said probe member (16) to the stenosis (26) to incise and to dilate the stenosis (26) in response to a distal movement of said moving means.

3. A device according to claim 1 or 2 wherein said means for reciprocally moving said probe member (16) and said incising means is a probe catheter (14) having a distal end (18).

4. A device according to any one of the preceding claims wherein said probe member (16) is fixedly attached to said distal end (18) of said probe catheter (14).

5. A device according to claim 4 when dependent on claim 2 wherein said probe catheter (14) and said probe member (16) are formed with a contiguous lumen (22) and said means for advancing is a guidewire (24), said guidewire (24) being prepositionable in the vessel and insertable through said lumen (22) to guide said probe member (16) to the stenosis.

6. A device according to claim 4 when dependent on claim 2 wherein said means for advancing is a guide catheter (32), said guide catheter (32) being prepositionable in the vessel and formed with a lumen (34) for receiving said probe member (16) and said probe catheter (14) therethrough to guide said probe member (16) to the stenosis (26).

7. A device according to any one of claims 4 to 6 wherein said probe member (16) is substantially cone-shaped with a tapered portion having a diminishing cross sectional area in the distal direction.

8. A device according to any one of claims 4 to 7 wherein said probe catheter (14) defines a longitudinal axis and said blades (20) are aligned longitudinally on said probe member (16).

9. A device according to any one of claims 4 to 8 further comprising a selectively inflatable balloon (38) mounted on said probe catheter (14) proximal to said probe member (16).

10. A device according to any one of claims 4 to 9 further comprising means for rigidizing (46) a portion of said probe catheter (14) proximal to said probe member (16) to facilitate movement of said probe member (16) through the stenosis (26).

## Patentansprüche

1. Vorrichtung zum Abtragen bzw. Ausräumen einer Stenose in einem Blutgefäß eines Patienten, die umfaßt:
ein Mittel zum Inzidieren der Stenose (26),
ein Sondenelement (16) zum Aufweiten der Stenose (26), und
ein Mittel zum Hin- und Herbewegen des Sondenelements (16) und des Inzisionsmittels durch die Stenose (26), um die Stenose (26) zu inzidieren und aufzuweiten, wobei das Sondenelement (16) fest an dem Bewegungsmittel angebracht ist,
dadurch gekennzeichnet, daß
das Inzisionsmittel eine Anzahl fest an dem Sondenelement (16) angebrachter und sich im wesentlichen distal von diesem erstreckender Klingen bzw. Schneiden (20) ist.

2. Vorrichtung nach Anspruch 1, ferner ein Mittel zum Vorwärtsbewegen des Inzisionsmittels und des Sondenelements (16) zu der Stenose (26) umfassend, um die Stenose (26) als Reaktion auf eine distale Bewegung des Bewegungsmittels zu inzidieren und aufzuweiten.

3. Vorrichtung nach Anspruch 1 oder 2, wobei das Mittel zum Hin- und Herbewegen des Sondenelements (16) und des Inzisionsmittels ein Sondenkatheter (14) mit einem distalen Ende (18) ist.

4. Vorrichtung nach einem der vorangehenden Ansprüche, wobei das Sondenelement (16) fest am distalen Ende (18) des Sondenkatheters (14) angebracht ist.

5. Vorrichtung nach Anspruch 4 in Abhängigkeit von Anspruch 2, wobei der Sondenkatheter (14) und das Sondenelement (16) mit einem angrenzenden Lumen (22) ausgebildet sind und das Mittel zum Vorwärtsbewegen ein Führungsdraht (24) ist, wobei der Führungsdraht (24) im Blutgefäß vorpositionierbar und durch das Lumen (22) einführbar ist, um das Sondenelement (16) zu der Stenose zu führen.

6. Vorrichtung nach Anspruch 4 in Abhängigkeit von Anspruch 2, wobei das Mittel zum Vorwärtsbewegen ein Führungskatheter (32) ist, der Führungskatheter (32) in dem Blutgefäß vorpositionierbar und mit einem Lumen (34) zur Aufnahme des Sondenelements (16) und des Sondenkatheters (14) durch dieses hindurch ausgebildet ist, um das Sondenelement (16) zu der Stenose (26) zu führen.

7. Vorrichtung nach einem der Ansprüche 4 bis 6, wobei das Sondenelement (16) im wesentlichen konusförmig mit einem sich verjüngenden Abschnitt mit in distaler Richtung abnehmender Querschnittsfläche ist.

8. Vorrichtung nach einem der Ansprüche 4 bis 7, wobei der Sondenkatheter (14) eine Längsachse festlegt und die Klingen bzw. Schneiden (20) in Längsrichtung am Sondenelement (16) ausgerichtet sind.

9. Vorrichtung nach einem der Ansprüche 4 bis 8, ferner einen selektiv aufblasbaren Ballon (38) umfassend, der am Sondenkatheter (14) proximal zum Sondenelement (16) angebracht ist.

10. Vorrichtung nach einem der Ansprüche 4 bis 9, ferner ein Mittel (46) zum Versteifen eines Abschnitts des Sondenkatheters (14) proximal zum Sondenelement (16) umfassend, um die Bewegung des Sondenelements (16) durch die Stenose (26) zu erleichtern.

## Revendications

1. Dispositif d'élimination d'une sténose dans un vaisseau d'un patient, qui comprend :
des moyens pour inciser la sténose (26) ;
un organe de sonde (16) pour dilater la sténose (26) ; et
des moyens pour déplacer en va-et-vient l'organe de sonde (16) et lesdits moyens d'incision à travers la sténose (26) pour inciser et dilater la sténose (26), l'organe de sonde (16) étant attaché fixement aux moyens de déplacement ; caractérisé en ce que lesdits moyens d'incision sont constitués d'une pluralité de lames (20) attachées fixement audit organe de sonde (16) et s'étendant sensiblement dans la direction distale depuis celui-ci.

2. Dispositif selon la revendication 1, comprenant en outre des moyens pour pousser lesdits moyens d'incision et ledit organe de sonde (16) vers la sténose (26) afin d'inciser et de dilater la sténose (26) en réponse à un mouvement distal desdits moyens de déplacement.

3. Dispositif selon la revendication 1 ou 2, dans lequel lesdits moyens de déplacement en va-et-vient dudit organe de sonde (16) et desdits moyens d'incision sont constitués par un cathéter à sonde (14) possédant une extrémité distale (18).

4. Dispositif selon l'une quelconque des revendications précédentes, dans lequel ledit organe de sonde (16) est attaché fixement à ladite extrémité distale (18) dudit cathéter à sonde (14).

5. Dispositif selon la revendication 4, lorsqu'elle dépend de la revendication 2, dans lequel ledit cathéter à sonde (14) et ledit organe de sonde (16) sont pourvus d'une lumière contiguë (22) et lesdits moyens de poussée sont constitués par un guide souple (24), ledit guide souple (24) étant prépositionnable dans le vaisseau et pouvant être inséré à travers ladite lumière (22) pour guider ledit organe de sonde (16) vers la sténose.

6. Dispositif selon la revendication 4, lorsqu'elle dépend de la revendication 2, dans lequel lesdits moyens de poussée sont constitués par un cathéter guide (32), ledit cathéter guide (32) étant prépositionnable dans le vaisseau et pourvu d'une lumière (34) destinée à recevoir ledit organe de sonde (16) et ledit cathéter à sonde (14) à travers afin de guider ledit organe de sonde (16) vers la sténose.

7. Dispositif selon l'une quelconque des revendications 4 à 6, dans lequel ledit organe de sonde (16) est sensiblement conique, avec une partie amincie présentant une zone de section transversale qui se réduit dans la direction distale.

8. Dispositif selon l'une quelconque des revendications 4 à 7, dans lequel ledit cathéter à sonde (14) définit un axe longitudinal et lesdites lames (20) sont alignées longitudinalement sur ledit organe de sonde (16).

9. Dispositif selon l'une quelconque des revendications 4 à 8, comprenant en outre un ballon gonflable de manière sélective (38) monté sur ledit cathéter à sonde (14) à proximité dudit organe de sonde (16).

10. Dispositif selon l'une quelconque des revendications 4 à 9, comprenant en outre des moyens pour rigidifier (46) une partie dudit cathéter à sonde (14) à proximité de l'organe de sonde (16) afin de faciliter le déplacement dudit organe de sonde (16) à travers la sténose (26).
